# EUROPÄISCHE PATENTSCHRIFT

(11) **EP 1 507 488 B1**
(45) Veröffentlichungstag und Bekanntmachung des Hinweises auf die Patenterteilung: **24.09.2008**
(21) Anmeldenummer: 03752711.6
(22) Anmeldetag: 18.02.2003
(51) Int. Cl.: A61B 19/00, A61B 5/103

(54) **ANORDNUNG ZUR ERMITTLUNG FUNKTIONSBESTIMMENDER GEOMETRISCHER GRÖSSEN EINES GELENKES EINES WIRBELTIERS**
ARRANGEMENT FOR DETERMINING FUNCTION-DETERMINED GEOMETRIC VARIABLES OF A JOINT OF A VERTEBRATE
ENSEMBLE PERMETTANT DE DETERMINER DES DIMENSIONS GEOMETRIQUES, A DEFINITION DE FONCTION, D'UNE ARTICULATION D'UN VERTEBRE

(30) Priorität: 21.05.2002 DE 10222416
(43) Veröffentlichungstag der Anmeldung: 23.02.2005
(73) Patentinhaber: Plus Orthopedics AG, 6343 Rotkreuz (CH)
(72) Erfinder: STIFTER, Jan, CH-5424 Unterehrendingen (CH); BROERS, Holger, 26670 Uplengen (DE)
(74) Vertreter: Popp, Eugen
(86) Internationale Anmeldenummer: PCT/EP2003/001635
(87) Internationale Veröffentlichungsnummer: WO 2003/096920

(56) Entgegenhaltungen:
- WO-A-02/17798
- US-A- 5 806 518
- US-A1- 2002 055 679

## Beschreibung

Die Erfindung betrifft eine Anordnung zur Ermittlung von funktionsbestimmenden geometrischer Größen eines Gelenkes eines Wirbeltiers.

Chirurgische Eingriffe zur Ersetzung von Gelenken oder Gelenkbestandteilen beim Menschen sind seit langem bekannt und gehören in den Industrieländern zum klinischen Alltag. Es gibt auch seit Jahrzehnten eine intensive Entwicklungsarbeit zur Bereitstellung und fortlaufenden Verbesserung entsprechender Implantate, insbesondere von Hüftgelenksimplantaten, zunehmend aber auch von Knie-, Schulter- und Ellenbogengelenkimplantaten sowie auch von Wirbelkörperersatzimplantaten. Parallel zu diesen Entwicklungen, die inzwischen zu einer fast unübersehbaren Vielfalt von derartigen Implantatkonstruktionen geführt haben, werden auch geeignete Operationstechniken und -hilfsmittel bereitgestellt und fortentwickelt, insbesondere auch auf die jeweiligen Implantatkonstruktionen angepasste Werkzeuge zum Einsetzen derselben.

Es versteht sich auch, dass Gelenkersatzoperationen die Gewinnung geeigneter Abbildungen des jeweiligen Gelenkbereiches vorangeht, auf deren Grundlage der Operateur ein geeignetes Implantat und seine Operationstechnik festlegt. Wurden hierzu früher zumeist Röntgenaufnahmen verwendet, sind in den letzten Jahren auch Computertomogramme zum Handwerkszeug des Operateurs geworden. Dessen ungeachtet ist der dauerhafte Erfolg von Gelenkersatzimplantationen auch heute noch eng mit der Erfahrung des Operateurs korreliert, was zu einem beträchtlichen Teil auf die nicht zu unterschätzenden Schwierigkeiten der adäquaten intraoperativen Nutzung bildlicher Darstellungen zur optimalen Ausrichtung der Teile des Gelenkimplantates in Bezug auf die effektiven Gelenkzentren und Belastungsachsen des einzelnen Patienten zurückzuführen ist.

Es hat daher in den letzten Jahren verstärkt Bemühungen zur Bereitstellung entsprechender Positionierungs-Hilfsmittel und -Verfahren für den Operateur gegeben, die im wesentlichen aus Entwicklungen auf dem Gebiet der Roboter- und Handhabungstechnik abgeleitet sind.

Die EP 0 553 266 B1 oder US 5,198,877 beschreiben ein Verfahren und ein Gerät zur kontaktlosen dreidimensionalen Gestaltdetektierung, das Anregungen zur Entwicklung medizinischer "Navigations"systeme und -verfahren gegeben hat; vgl. auch die ausführlichen Literaturhinweise in diesen Druckschriften.

Aus der US 5,871,018 und der US 5,682,886 sind Verfahren zur Ermittlung der Belastungsachse des Femurs bekannt. Gemäß diesen Verfahren werden in einem ersten Schritt die Koordinaten des Femurs beispielsweise durch eine Computertomographieaufnahme ermittelt und in einem Computer abgespeichert. Mit Hilfe der abgespeicherten Daten wird dann ein dreidimensionales Computermodell des Femurs erstellt, und anhand dieses Modells werden die optimalen Koordinaten für das Ansetzen einer Lehre an den Knochen sowie einer anschließend einzusetzenden Knieprothese berechnet. Grundlage hierfür ist die Berechnung der Belastungsachse des Femurs.

Nach einer derartigen Simulation wird der Femur des Patienten fixiert, und mit einer Registrierungseinrichtung werden einzelne Punkte an der Femuroberfläche abgetastet, um die Orientierung des Femurs für die durchzuführende Operation festzustellen. Dieses Abtasten des Knochens erfordert, dass entweder große Teile längs des Femurs möglichst bis zum Hüftgelenk hin offengelegt sein müssen, um deren Oberfläche mit der Registrierungseinrichtung abtasten zu können, oder eine Art Nadel zum Durchstechen der Haut bis auf den Knochen als Abtastinstrument benutzt wird. Da jedoch jeder operative Eingriff für den Patienten ein Risiko darstellt und Nadelstiche Blutergüsse und ein zusätzliches Infektionsrisiko in den Knochenpartien verursachen, ist es nicht wünschenswert, einen zusätzlichen operativen Eingriff im Bereich der Hüfte vorzunehmen oder Nadeleinstiche entlang des Femurs durchzuführen, um den Ort des Rotationszentrums festzustellen. Des weiteren ist eine strenge Fixierung des Femurs auf einem Messtisch der Registrierungseinrichtung notwendig, da ansonsten Verschiebungen der Hüftpfanne während der Antastprozedur auftreten und die Schnittlehre nach erfolgter Registrierung der Femurkoordinaten falsch angesetzt werden würde.

Die FR 2 785 517 beschreibt ein Verfahren und eine Vorrichtung zum Detektieren des Rotationszentrums des Femurkopfes in der Hüftpfanne. Hierfür wird der Femur mit seinem Femurkopf in der Hüftpfanne bewegt, und die in verschiedenen Stellungen des Femurs aufgenommenen Messpunktkoordinaten werden abgespeichert. Sobald eine Verschiebung des Rotationszentrums des Femurs auftritt, wird ein entsprechender Gegendruck auf den Femurkopf ausgeübt, der bei der Bestimmung eines Punktes, der in Beziehung mit der Anordnung des Femurs steht, mit berücksichtigt wird.

In der DE 197 09 960 A1 werden ein Verfahren und eine Vorrichtung zur präoperativen Bestimmung der Positionsdaten von Endoprothesenteilen eines mittleren Gelenkes relativ zu den das mittlere Gelenk ausbildenden Knochen beschrieben. Hierbei wird vorgeschlagen, dass man durch Bewegung der Knochen um jeweils ein äußeres Gelenk, das sich an dem dem mittleren Gelenk abgewandten Ende des jeweiligen Knochens befindet, jeweils einen äußeren Gelenkpunkt bestimmt, dass man im Bereich des besagten mittleren Gelenkes für jeden der beiden Knochen ebenfalls einen Gelenkpunkt bestimmt, dass man durch geradlinige Verbindung der beiden so gefundenen Gelenkpunkte für jeden der beiden Knochen eine für diesen charakteristische Richtung bestimmt und schließlich die Orientierung der Endoprothesenteile relativ zu dieser charakteristischen Richtung bestimmt.

Ähnliche medizinische "Navigations"verfahren werden in der WO 95/00075 und der WO 99/23956 beschrieben, wobei Bilderfassungssysteme der oben erwähnten Art zur Positionserfassung von Referenzen an den zum jeweiligen Gelenk benachbarten Knochen eingesetzt werden und aus der mit ihrer Hilfe gewonnenen virtuellen Repräsentation des Knochens bzw. Gelenkes charakteristische Punkte bzw. Achsen abgeleitet werden können.

Ein hinsichtlich der Zuverlässigkeit und insbesondere der Unabhängigkeit von intraoperativen Bewegungen des Patienten verbessertes und für den unmittelbaren Einsatz während der Operation, insbesondere der Implantation eines künstlichen Kniegelenkes, vorgesehenes System dieser Art ist Gegenstand der auf die Anmelderin zurückgehenden WO 02/17798 A1.

Diese Druckschrift lehrt eine Anordnung zur Ermittlung der Belastungsachse einer Extremität eines Menschen, zur Vorbereitung des Einsetzens eines Gelenkersatzimplantats, insbesondere einer Hüft- oder Schultergelenkpfanne oder eines zugehörigen Schaftimplantats, mittels eines optischen Koordinatenmessverfahrens, mit
- einer Stereokamera oder -kameraanordnung zur räumlichen Erfassung optischer Gebersignale, - einem mobilen Mehrpunktgeber, der als beweglicher Taster zum Antasten knöcherner Referenzen im Gelenkbereich zur Bestimmung von deren Koordinaten ausgebildet ist,
- einem skelettfesten Mehrpunktgeber, der zur starren Befestigung, insbesondere zum Anschrauben oder Anklemmen, an einer vom dem Gelenk ausgehenden Extremität, insbesondere nahe dem proximalen Ende eines Femurs oder eines Humerus, ausgebildet ist,
- einer Ablaufsteuerung zur Steuerung der sequentiellen Registrierung und Abspeicherung von einer durch den mobilen Mehrpunktgeber gelieferten Messpunktkoordinatenmengen und von in einer ersten Mehrzahl von Positionen des skelettfesten Mehrpunktgebers in einer Mehrzahl von Drehstellungen der Extremität erfassten Messpunktkoordinatenmengen und deren anschließenden Verarbeitung nach einem vorgespeicherten Verarbeitungsablauf,
- einer Auswertungseinheit zur Auswertung der durch die Mehrpunktgeber gelieferten und die Kameraanordnung erfassten Messpunktkoordinatenmengen zwecks Bestimmung der Belastungsachse mittels einer Ausgleichungsrechnung nach der Methode der kleinsten Quadrate, und
- einer mit der Ablaufsteuerung sowie der Auswertungseinheit verbundenen Ausgabeeinheit zur Ausgabe von Handlungsaufforderungen an einen Operateur gemäß dem vorbestimmten Verfahrensablauf sowie zur Anzeige der Auswertungsergebnisse.

Der Erfindung liegt, ausgehend vom Stand der Technik, die Aufgabe der Bereitstellung einer für den Operateur schnell und einfach sowie mit sehr geringem Fehlerrisiko bedienbaren Anordnung dieser Art zugrunde, die die Erzielung deutlich verbesserter Operationsergebnisse speziell für Hüft- und Schultergelenkimplantate ermöglicht.

Diese Aufgabe wird durch eine Anordnung mit den Merkmalen des Anspruchs 1 gelöst. Zweckmäßige Ausgestaltungen des Erfindungsgedankens sind Gegenstand der Unteransprüche. Deren Gegenstände liegen - in beliebiger Kombination miteinander - auch in modifizierten Ausführungen im Bereich der vorliegenden Erfindung.

Ein wesentlicher Gedanke der Erfindung besteht darin, die vorgeschlagene Anordnung zur Ermittlung funktionsbestimmender geometrischer Größen eines Gelenkes in Vorbereitung einer Gelenkersatzimplantation mit einer Stereokamera oder -kameraanordnung und zwei verschiedenen Arten von Signalgebern für diese auszuführen. Es handelt sich hierbei um (mindestens) einen ersten ("mobilen") Mehrpunktgeber, der als beweglicher Taster zum Antasten knöcherner Referenzen im Gelenkbereich zur Bestimmung von deren Koordinaten ausgebildet ist, und einen zweiten ("skelettfesten") Mehrpunktgeber, der zur starren Befestigung, insbesondere zum Anschrauben oder Anklemmen, in einem vom Gelenk hinreichend beanstandeten Abschnitt einer vom dem Gelenk ausgehenden Extremität, insbesondere nahe dem proximalen oder distalen Ende eines Femurs oder eines Humerus, ausgebildet ist.

Weiterhin schließt die Erfindung den Gedanken des Vorsehens einer interaktiven Ablaufsteuerung zur Steuerung der sequentiellen Registrierung und Abspeicherung von in einer ersten Mehrzahl von Tasterpositionen des ersten Mehrpunktgebers und einer zweiten Mehrzahl von Drehstellungen der Extremität erfassten Messpunktkoordinatenmengen und deren anschließenden Verarbeitung nach einem vorbestimmten Verarbeitungsablauf ein.

Schließlich gehört zur Erfindung der Gedanke des Vorsehens einer geeignet ausgebildeten Auswertungseinheit zur Auswertung der durch die Mehrpunktgeber gelieferten und die Kameraanordnung erfassten Messpunktkoordinatenmenge zwecks Bestimmung der geometrischen Größen. Diese Auswertungseinheit umfasst die Mittel zur Bestimmung der transversalen, vertikalen und sagittalen Körperebenen und -achsen sowie Mittel zur Ausführung eines iterativen Verfahrens, insbesondere einer Ausgleichungsrechnung nach der Methode der kleinsten Quadrate, zur Bestimmung der Koordinaten des Rotationszentrum des Gelenkes.

Letztlich gehört zur erfindungsgemäßen Anordnung eine mit der Ablaufsteuerung sowie der Auswertungseinheit verbundene Ausgabeeinheit zur Ausgabe von Handlungsaufforderungen an einen Operateur gemäß dem vorbestimmten Verfahrensablauf und in Abhängigkeit von den Ergebnissen der Bestimmung der geometrischen Größen sowie zur Anzeige der Auswertungsergebnisse.

Zweckmäßigerweise ist die erwähnte Ausgabeeinheit zur Anzeige der Auswertungsergebnisse in grafischer Darstellung, insbesondere in einer synoptischen bildlichen Darstellung mit einem durch ein bildgebendes Untersuchungsverfahren gewonnenen zwei- oder dreidimensionalen Abbild des Gelenkbereiches, ausgebildet. Dem Operateur wird hierdurch - unabhängig von einer im System vorteilhafter Weise implementierten interaktiven Benutzerführung und automatischen Steuerungsfunktionen - eine gute Möglichkeit zur Gewinnung eines visuellen Eindrucks von den geometrischen Verhältnissen im Gelenkbereich und gegebenenfalls der Lage eines Werkzeugs oder des Implantates relativ zu diesem gegeben.

Speziell für eine Pfannenimplantation (im Hüft- wie Schulterbereich) wird noch ein weiterer skelettfester Mehrpunktgeber eingesetzt, welcher an einem knöchernen Bereich auf der Pfannenseite des Gelenkes (auf etwa am Beckenkamm) fest angebracht wird und dessen Positionssignale in Verbindung mit denen des mobilen Tasters zur pfannenseitigen Positionsbestimmung dienen.

Wesentlich für einen breiten praktischen Einsatz der vorgeschlagen Anordnung ist eine mit der Ablaufsteuerung und der Auswertungseinheit verbundene Eingabeschnittstelle zur Eingabe von Lagebeziehungsvektoren zwischen definierten realen oder virtuellen Punkten des Gelenkbereiches und/oder von Lagebeziehungsvektoren zwischen solchen Punkten innerhalb des Gelenkbereiches oder von diesen zu gelenkfunktionsrelevanten Punkten an der Extremität außerhalb des Gelenkbereiches und/oder von Implantatparametern einer vorbestimmten Menge von einsetzbaren Gelenkersatzimplantaten oder zur Vorgabe möglicher Implantatpositionen und -ausrichtungen. Es handelt sich hierbei entweder um eine als Benutzerschnittstelle zur Tastatur- oder Spracheingabe von Daten durch den Operateur oder eine zur Übernahme von Daten aus einem auf einem bildgebenden Untersuchungsverfahren beruhenden Auswertungsprogramm oder einer diese Funktionen miteinander kombinierende Schnittstelle.

Zweckmäßigerweise gehört zur Anordnung mindestens eine verstellbare Klemmvorrichtung als Adapter zur Fixierung des skelettfesten Mehrpunktgebers an der Extremität oder der Mehrpunktgeber an Extremität und Gelenk, oder eine adäquate Befestigungsvorrichtung auf der Basis von in Knochen verankerten Schrauben oder Nägeln.

Weiterhin ist der mobile Mehrpunktgeber zum äußeren Antasten von knöchernen Referenzen an der von dem zu ersetzenden Gelenk und wahlweise dem zweiten Hüft- oder Schultergelenk ausgehenden zweiten Extremität ausgebildet oder hierzu ein weiterer, als beweglicher Taster ausgebildeter Mehrpunktgeber zum Antasten derartiger Referenzen vorgesehen. Dann ist die Auswertungseinheit zur Auswertung der Messpunktkoordinaten dieser knöchernen Referenzen zur Bestimmung mindestens einer der geometrischen Größen, insbesondere der Länge der Extremität, ausgebildet.

Sinnvoll ist überdies eine Ergänzung der Anordnung durch einen dritten (skelettfesten) Mehrpunktgeber zur im wesentlichen starren Befestigung, insbesondere mittels einer verstellbaren Manschette, an einer zweiten Extremität, die von einem nicht zu operierenden zweiten Hüft- oder Schultergelenk ausgeht. Hierbei ermöglicht dann die Auswertungseinheit die Bestimmung von geometrischen Größen des zweiten Hüft- bzw. Schultergelenkes als Referenz für die geometrischen Größen des ersten Gelenkes ausgebildet.

Eine integrierte Gesamtanordnung der erfindungsgemäßen Art umfasst bevorzugt auch ein Resektionsinstrument, insbesondere einen Fräser oder eine Raspel, zur Ausformung des Implantationsbereiches und/oder ein navigierbares Setzinstrument, insbesondere Schraubwerkzeug, zur Montage des Gelenkersatzimplantates. In Zuordnung zu einem von diesen oder zu beiden Werkzeugen ist ein weiterer Mehrpunktgeber vorgesehen oder es wird der o.a. mobile Mehrpunktgeber hiermit eingesetzt. Dieser ist mit dem jeweiligen Werkzeug starr zu einer geometrisch kalibrierten, navigierbaren Werkzeug-Geber-Einheit verbindbar, derart, dass aus den Gebersignalen dieser Einheit Positionskoordinaten eines Wirkabschnitts des Instruments, und hieraus wahlweise Positionskoordinaten eines mit dem Resektionsinstrument geschaffenen Resektionsabschnittes bzw. des Implantates bestimmbar sind. Hierbei ist die Eingabeschnittstelle speziell zur Eingabe von Instrumentparametern des Resektionsinstrumentes und/oder von Werkzeugparametern des Setzinstruments ausgebildet.

In einer weiteren sinnvollen Fortbildung des Erfindungsgedankens umfasst die Anordnung eine Sonde, insbesondere Markkanalahle, zur Sondierung eines Markkanals der von dem Gelenk ausgehenden Extremität, die mit einem Mehrpunktgeber starr zu einer geometrisch kalibrierten, navigierbaren Sonden-Geber-Einheit verbindbar ist, derart, dass aus den Geber-Signalen dieser Einheit ein Richtungsvektor des Markkanals bestimmbar ist. Es versteht sich, dass hier die Eingabeschnittstelle zur Eingabe von Sondenparametern geeignet sein muss.

Der oder die Mehrpunktgeber ist/sind vorzugsweise als passiver Vierpunktgeber mit vier Kungelreflektorabschnitten ausgebildet. Der Stereokamera bzw. Kameraanordnung ist hierbei eine Beleuchtungseinrichtung zugeordnet, mit der der oder die Mehrpunktgeber angeleuchtet werden, so dass definierte Reflexe zur "Abbildung" des jeweiligen Mehrpunktgebers zur Verfügung stehen. Zur Vermeidung von den Operateur störenden Lichtreflexen arbeitet die Beleuchtungseinrichtung bevorzugt im Infrarotbereich.

Eine den Operateur besonders weitgehend unterstützende Ausführung der vorgeschlagenen Anordnung umfasst eine mit der Auswertungseinheit und der Matching-Verarbeitungseinheit verbundene Steuersignal-Erzeugungseinheit. Diese ist die zu einem Vergleich eines Satzes von über die Eingabeschnittstelle eingegebenen und an die Realpositionskoordinaten des Gelenk- bzw. Wirbelbereiches angepassten Implantatpositionsdaten bzw. -Ausrichtungsdaten mit aktuell erfassten Realpositionskoordinaten des Wirkabschnitts des Resektions- oder Setzinstruments und zur Bestimmung einer Abweichung zwischen Soll- und Ist-Positionskoordinaten und zur Ausgabe von Abweichungsdaten oder eines aus der Abweichung abgeleiteten Steuerbefehls, insbesondere per Text- oder Sprachausgabe und/oder in einer synoptischen Darstellung mit dem Abbild, ausgebildet.

Vorteile und Zweckmäßigkeiten ergeben sich im übrigen aus der nachfolgenden Beschreibung eines bevorzugten Ausführungsbeispiels - einer Anordnung in Verbindung mit einem Verfahren zur Implantation eines künstlichen Hüftgelenkes - in Verbindung mit den Figuren. Von diesen zeigen:
- Fig. 1: einen Beckenkamm-Lokator mit zugehöriger Klammer (Adapter), aufgeklemmt auf einen Beckenkamm, in perspektivischer Darstellung,
- Fig. 2: zusätzlich einen Handtaster zum Antasten der Tischoberfläche zwecks Bestimmung der Tischebene sowie knöchener Referenzen am Beckenkamm (über der Haut), in perspektivischer Darstellung,
- Fig. 3: zusätzlich zum Beckenkamm-Lokator eine perspektivische Darstellung eines Femur-Lokators mit zugehöriger Klammer zur Fixierung im proximalen Bereich eines Femurs,
- Fig. 4: eine Kugeladapter-Handtaster-Kombination zur Bestimmung des Zentrums des Acetabulums in perspektivischer Darstellung,
- Fig. 5: eine perspektivische Darstellung einer Fräser-Lokator-Kombination zum Fräsen des Sitzes für eine Hüftpfanne,
- Fig. 6: eine schematische Ausschnittdarstellung eines Schirmbildes eines PC-Monitors zur visuellen Darstellung der Aussichten des Fräsers bezüglich des Beckens,
- Fig. 7: eine perspektivische Darstellung einer Setzinstrument-Lokator-Kombination zum Eindrehen einer künstlichen Hüftpfanne in den vorbereiteten Sitz und
- Fig. 8: eine perspektivische Darstellung einer Markkanalahlen-Lokator-Kombination zur Bestimmung des Verlaufes des Markkanals in einem Femur.

Die nachfolgende Darstellung wird primär anhand eines Vorgehens zur Bestimmung der relevanten geometrischen Größen sowie zur Implantation einer Hüftpfanne gegeben, ergänzend wird aber auch auf die - hiervon relativ unabhängige - Bestimmung der relevanten geometrischen Größen und die Ausführung der Implantation eines Schaftteiles als zweiter Komponente eines künstlichen Hüftgelenkes hingewiesen.

Der Operateur hat in der Planung einer Hüftgelenkimplantation für die Pfanne folgende Werte zu bestimmen:
1. Größe der künstlichen Pfanne
2. Inklinationswinkel und Antetorsionswinkel
   Die beiden Winkel der Ausrichtung der Pfannenachse relativ zu den Körperebenen werden hier vom Operateur nach medizinischen Gesichtspunkten im Röntgenbild gewählt. Diese Winkel können ebenfalls vom Operateur intraoperativ verändert werden.
3. Winkel in der sagittalen Körperebene zwischen vertikaler Achse und der Richtung vom Beckenkamm zur Symphyse.
   Durch die Bestimmung dieses Winkels wird eine intraoperative Bestimmung der Körperachsen und damit des Planungskoordinatensystems ermöglicht.

Es wird angenommen, dass der Patient sich zu Beginn der Operation in Rückenlage befindet; der Arzt hat ein Röntgenbild zur Verfügung, welches hinreichend die anatomische Gesamtkonstellation sowie die Knochenbeschaffenheit erkennen lässt. Er gewinnt daraus erste Vorstellungen hinsichtlich der einzusetzenden Implantatgröße und der zu bevorzugenden Grobausrichtung des Implantates. Es wird eine Inzision 3 - 5 cm dorsal der Spina Iliaca Superior Anterior mit einer Länge von 4 cm vorgenommen, der Beckenkamm freigelegt und das Gewebe mit dem Raspatorium freigelegt.

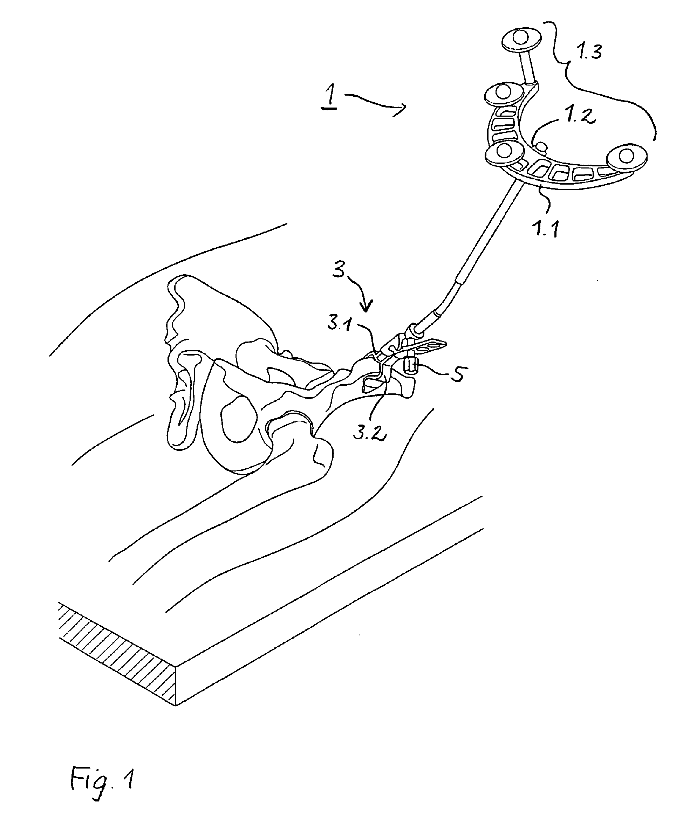

Fig. 1 zeigt einen Beckenkamm-Lokator 1 mit zugehöriger Befestigungsklammer 3, der im freigelegten Bereich des Beckenkammes angebracht wird. Die Befestigungsklammer 3 umfasst ein mediales Klammerteil 3.1 und ein laterales Klammerteil 3.2, die über eine Inbusschraube 5 miteinander verschraubt werden, bis die Befestigungsklammer fest am Beckenkamm sitzt. Der eigentliche Beckenkamm-Lokator 1 hat einen sichelförmig geschwungenen Grundkörper 1.1 mit einer Steckhülse 1.2 zum Aufstecken auf die Befestigungsklammer 3 sowie einem 4-Punkt-Lokatorfeld 1.3 aus vier IR-reflektierenden Kugeln, die jeweils partiell von einem (nicht gesondert bezeichneten) kugelabschnittförmigen Diffusor zur Vermeidung von Störstrahlungseinflüssen umgeben sind. Es handelt sich hierbei um sogenannte passive Targets oder Adapter, die als solche bekannt sind und deren Wirkungsweise in Verbindung mit einer - ebenfalls bekannten - Stereokameraanordnung eines sogenannten Navigationssystems daher hier nicht weiter beschrieben wird. Nach dem Aufstecken wird der Lokator 1 gegenüber der Befestigungsklammer 3 derart gedreht, dass das Lokatorfeld zur Kamera geeignet ausgerichtet ist, ohne dass eine der reflektierenden Kugeln durch eine andere abgeschirmt ist. Anschließend wird durch Verschrauben des Lokators mit der Befestigungsklammer eine starre Verbindung zwischen beiden hergestellt.

Statt am Beckenkamm kann der oben als Beckenkamm-Lokator bezeichnete Mehrpunktgeber 1 auch am Pfannendach des Beckens befestigt werden. Dies hat den Vorteil, dass die oben erwähnte (zusätzliche) Inzision im Bereich des Beckenkammes überflüssig wird, jedoch ist die Befestigung des Mehrpunktgebers - der dann als "OP-Feld-Lokator" bezeichnet wird - bei geschwächter Knochenstruktur weniger stabil.

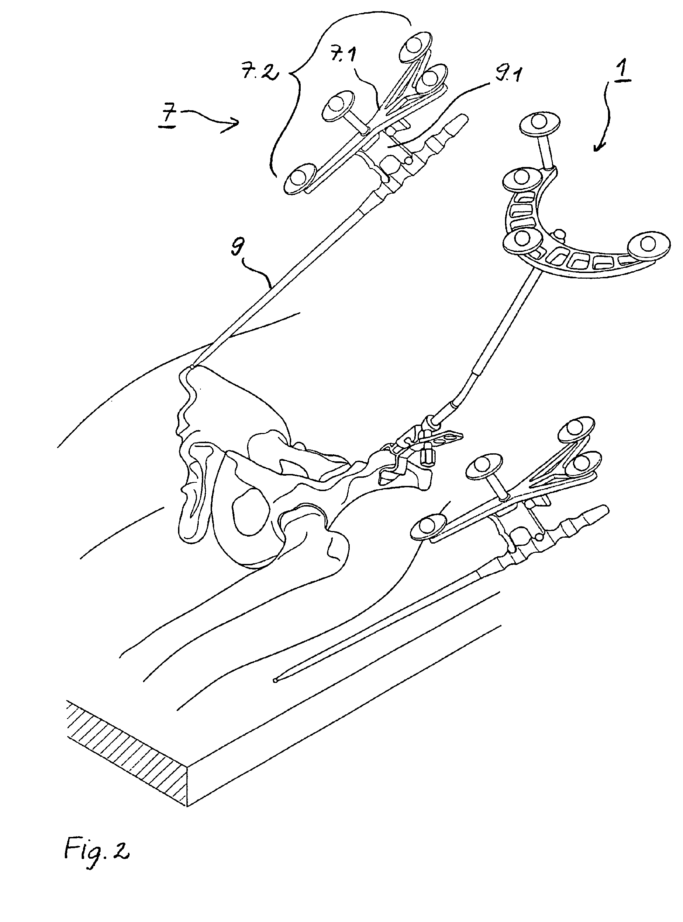

Fig. 2 zeigt neben dem oben beschriebenen skelettfesten Lokator 1 einen Handtaster 7 mit einem stabförmigen, sich zu einem Ende hin verjüngenden Tasterteil 9, von dem ein Halter 9.1 senkrecht absteht, einem annähernd Y-förmigen Taster-Grundkörper 7.1 und einem 4-Punkt-Lokatorfeld 7.2, ähnlich dem Aufbau des oben beschriebenen Beckenkamm-Lokators. Ähnlich sind auch die Lokatoren der weiter unten beschriebenen Anordnungskomponenten aufgebaut, so dass für diese die entsprechende Benennung der Teile bzw. Abschnitte und Beschreibung derselben fortgelassen wird.

Mit dem Handtaster 7 werden zu Beginn des Navigations-Ablaufes verschiedene Punkte der Ebene des OP-Tisches, auf dem der Patient liegt, angetastet, um eine Bestimmung der Lage der Tischebene im Raum zu ermöglichen. Diese wird zwar für die eigentliche Bestimmung der Patientenlagen nicht benötigt, kann aber für Plausibilitätsbetrachtungen (beispielsweise hinsichtlich der Bedeutung einer Beckenneigung des Patienten gegenüber der Tischebene etc.) herangezogen werden. Für die eigentliche Navigation wird üblicherweise angenommen, dass die Patienten-Frontalebene parallel zur Tischebene liegt.

Anschließend werden mit dem Handtaster 7 über der Haut charakteristische knöcherne Referenzen im Beckenbereich angetastet. Zunächst werden der linke und der rechte Beckenkamm sowie die Mitte der Symphyse angetastet. Durch diese drei angetasteten Punkte und den in der Planung ermittelten Kamm-Symphysen-Winkel können die Körperachsen eindeutig festgelegt werden. Die Richtung vom linken zum rechten Beckenkamm stellt die transversale Körperachse dar. Die Richtung von der Mitte der Beckenkammpunkte zur Symphyse wird um den Kamm-Symphysen-Winkel um die transversale Achse gedreht und stellt damit die vertikale Körperachse dar (orthogonal zur transversalen Achse). Die sagittale Körperachse ergibt sich aus den beiden erstgenannten Achsen als Orthogonale.

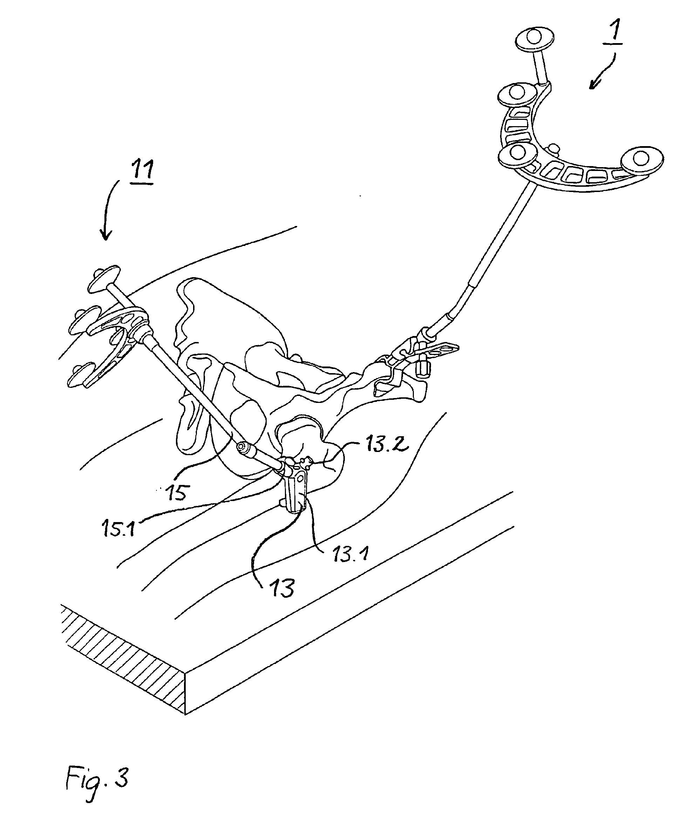

Fig. 3 zeigt zusätzlich zum Beckenkamm-Lokator 1 einen Femur-Lokator 11 mit zugehörigem Adapter (Femurklammer) 13 zur Befestigung nahe dem proximalen Ende des Femurs. Die Femurklammer 13 hat einen zweiteiligen Grundkörper aus einem in der Draufsicht gabelförmigen und in der Seitenansicht annähernd L-förmigen ersten Grundteil 13.1, von dem zwei Zapfen 13.2 zum Aufstecken des Lokators abstehen, und einem mit dem ersten Grundteil 13.1 verrastbaren, in der Seitenansicht annähernd L-förmiges zweiten Grundteil. Der Aufbau des Femur-Lokators 11 selbst entspricht - abgesehen von einem abgewinkelten Lokatorstab - im wesentlichen dem des Beckenkamm-Lokators.

Er wird über eine Steckhülse 15.1 am freien Ende eines Lokatorstabes 15 auf einen der beiden Zapfen 13.2 der Femurklammer 13 aufgesteckt.

Dann wird die Femurklammer 13 mit dem angesetzten Lokatorstab 15 auf der lateralen Femurseite etwa auf Höhe Trochanter Minor oder zwischen Trochanter Minor und Trochanter Major befestigt, indem die dort liegenden Muskelgruppen beiseite geschoben werden und die Klammer eingeschoben wird. Die Drehstellung ist so zu wählen, dass der Lokatorstab lateral aus dem OP-Feld, möglichst in Richtung der Kamera, ragt. Dann wird die Klammer mit mittlerem Drehmoment festgezogen, dass eigentliche (hier nicht gesondert bezeichnete) Lokatorfeld aufgesteckt und nach der Kamera ausgerichtet und schließlich der Femur-Lokator festgeschraubt.

Danach wird das kinematische Rotationszentrum der Hüfte sowohl im hüftfesten als auch im femurfesten Koordinatensystem durch mehrere relative Messungen des Femur-Lokators im hüftfesten Koordinatensystem bei unterschiedlichen Beinstellungen bestimmt. Die Transformation aller Messwerte kann somit vom hüftfesten Koordinatensystem in das Koordinatensystem der Körperachsen erfolgen. Hiermit können dann alle kalibrierten Werkzeuge zum Körperachsenkoordinatensystem ausgerichtet werden; siehe dazu weiter unten. Mit dem Rotationszentrum als Ursprung kann das Implantat an seinem kinematischen Ursprung eingesetzt werden. Sollten Korrekturen erforderlich sein, können Verschiebungen und Winkelveränderungen der Planung intraoperativ ausgeführt werden.

Nachdem der Operateur die Positionserfassungen in den verschiedenen Beinstellungen im "Dialog" mit der interaktiven Benutzerführung vorgenommen hat (wobei wieder eine Fehlerkorrektur aufgrund von Plausibilitätsberechnungen vorgesehen ist), wird der Femur-Lokator von der Klammer 13 abgenommen und der Femurkopf reseziert. Der Durchmesser des resezierten Kopfes wird gemessen und auf Grundlage des Messergebnisses eine geeignete Halbkugel für den nächsten Schritt, nämlich die Bestimmung des Zentrums des Acetabulums bzw. geometrischen Rotationszentrums der Hüfte, ausgewählt.

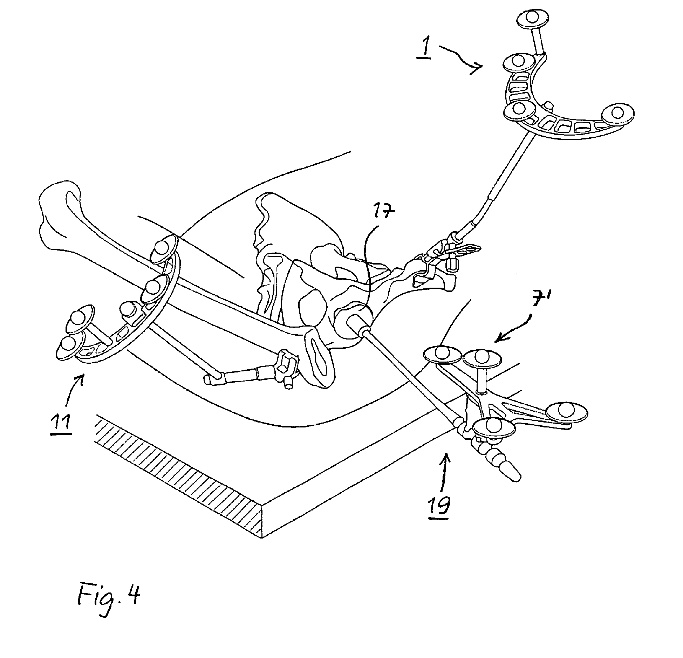

Wie Fig. 4 zeigt, wird die ausgewählte Halbkugel 17 mit einem Handtaster 7' der in Fig. 2 gezeigten und weiter oben beschriebenen Art zu einer Kugeladapter-Handtaster-Kombination 19 zusammengestellt. Durch Einsteuern dieses Lokators in den Pfannenbereich wird (üblicherweise unter Voraussetzung eines bestimmten Anteversionswinkels, z.B. 12°) zum einen die Gültigkeit des mittels des Femur-Lokators bestimmten (kinematischen) Rotationszentrums aus geometrischer Sicht überprüft, und zum anderen erlauben die Ergebnisse eine "Gegenprobe" zu der Implantations-Planungswerte unter geometrischen Gesichtspunkten. Außerdem lassen sich durch Bewegen der Halbkugel 17 im Pfannenbereich Hinweise auf mögliche mechanische Kollisionen gewinnen. Die Konstruktion der Halbschale und deren Adaption zum Handtaster realisiert die Antastspitze immer im Kugel-Zentrum der Antasthalbkugel.

Anschließend erfolgt im Rahmen des gespeicherten Auswertungsprogramms mit interaktiver Benutzerführung die endgültige Planung der Implantation, von der Bestimmung der einzusetzenden Implantatgröße bis hin zu Verschiebungswerten und Winkelgrößen. Das System errechnet auf dieser Grundlage sowie anhand von vorab eingegebenen spezifischen Instrumentdaten Sollpositionen für die einzusetzenden Resektions- bzw. Setzinstrumente oder, genauer gesagt, für deren Wirkabschnitte.

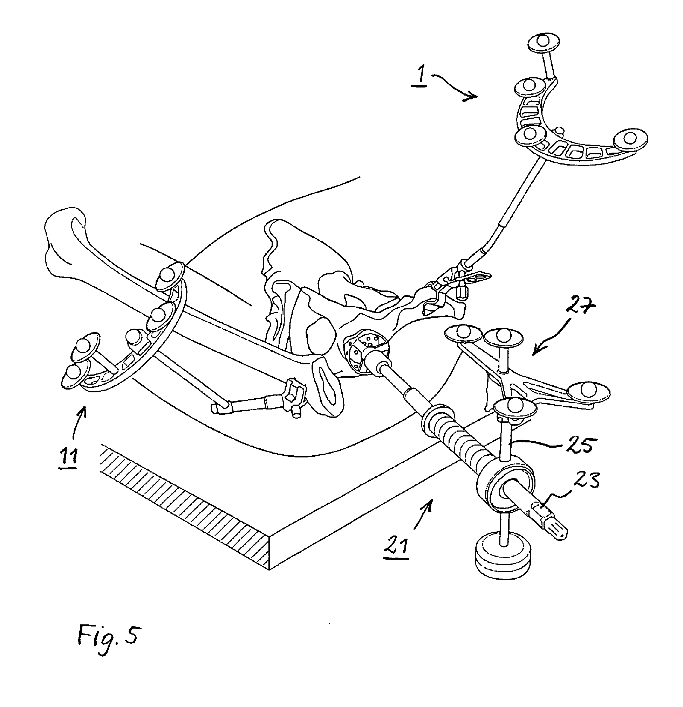

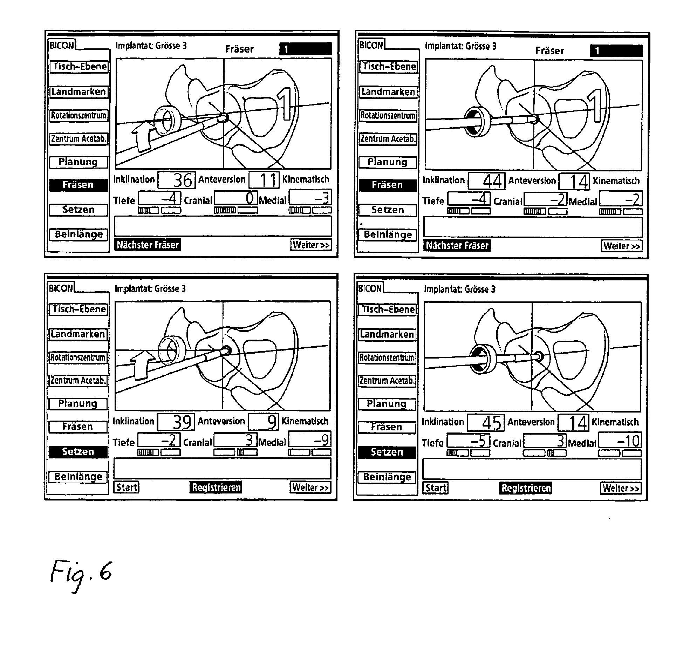

Fig. 5 zeigt neben Beckenkamm- und Femur-Lokator 1, 11 eine Fräser-Lokator-Kombination 21 mit einer Fräswelle 23, einem Fräswellenadapter 25 und einem Lokator 27, dessen Aufbau im wesentlichen denjenigen des Femur-Lokators 11 nach Fig. 3 entspricht. Dieses Instrument wird in der in der Figur ebenfalls gezeigten Weise in einem Pfannenbereich ausgerichtet, wobei die Lage und Ausrichtung aufgrund der Positionssignale aus dem Lokatorfeld erfasst und auf Schirmbildern in der in Fig. 6 gezeigten Weise visuell verdeutlicht wird. Eine entsprechend den Planungsdaten korrekte Lage des Fräsers wird durch einen um die Fräswelle sich erstreckenden Ring in der Displaydarstellung sowie durch akustische Signale angezeigt.

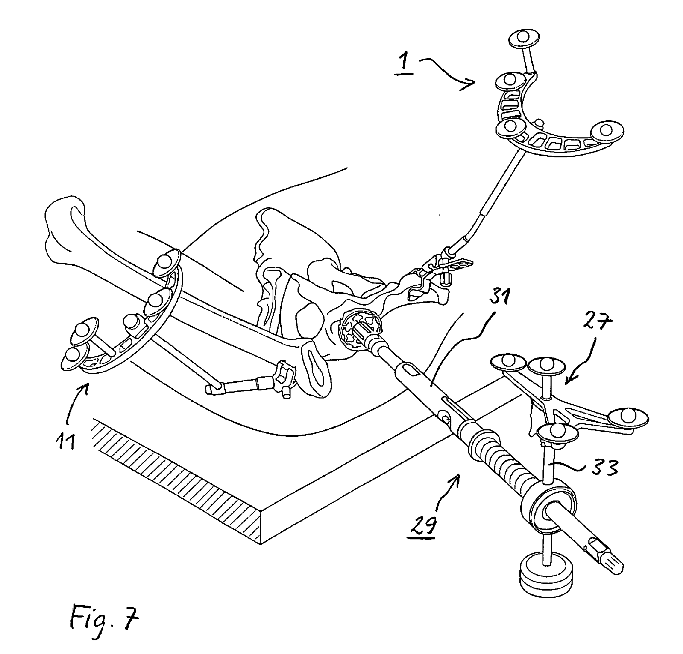

Sobald ein Pfannensitz gemäß den Planungsdaten hergestellt ist, wird die Fräser-Lokator-Kombination in eine Setzinstrument-Lokator-Kombination 29 umgebaut, wie sie in Fig 7 gezeigt ist. Hierbei wird wiederum der Lokator 27 eingesetzt, und zwar jetzt in Verbindung mit einem Setzinstrumentenschaft 31 und einem Schaftadapter 33. Mit diesem Instrument wird in einer zur Handhabung der Fräser-Lokator-Kombination weitgehend analogen und ebenso auf dem PC-Schirmbild dargestellten Art und Weise eine Hüftpfanne 35 gesetzt. Deren endgültige Position wird durch den Operateur noch in das System eingegeben.

Anschließend erfolgen die Schaftpräparation und -implantation (zunächst eines Testschaftes), und zwar entweder auf konventionelle oder ebenfalls durch das Navigationssystem gestützte Weise. Höhe und Anteversion des Schaftes sind dabei anhand der Planungsdaten fest eingestellt; lediglich die Kugelhalslänge ist noch frei wählbar. Dann wird das Gelenk mit dem Testschaft zusammengebaut und Stabilität sowie etwaige Kollisionen beim Bewegen des Schaftes in der Pfanne geprüft. Zudem erfolgt eine Grob-Prüfung der Beinlänge durch Vergleich der Lage der Knöchel des operierten des gesunden Beines. Falls Probleme bei der Gelenkstabilität auftreten, wird versucht, diese durch Wahl einer bestimmten Kugel oder eines Schaftes anderer Größe aus einem bereitstehenden Sortiment zu lösen.

Optional können in dieser Phase auch Messungen am anderen Bein unter Einsatz des Navigationssystem erfolgen, deren Ergebnisse im Sinne von Symmetriebetrachtungen zu einer Feinjustierung des Implantats herangezogen werden können. Er versteht sich, dass bei derartigen Messungen anstelle des oben beschriebenen Femur-Lokators ein zur äußeren Anbringung über der Haut modifizierter Femur-Lokator eingesetzt wird.

Ein wesentlicher Vorteil des vorgeschlagenen Systems besteht darin, dass unter Nutzung von Navigationsdaten auch ein Vorher-Nacher-Vergleich der Beinlängen (an der kranken Hüfte vor der Operation und während des oben erwähnten Überprüfungsschrittes in der Schlussphase der Operation) möglich ist. Hierzu wird der Femur-Lokator erneut auf dem am Femur verbliebenen Halter positioniert und fixiert und die Position bei gestrecktem, parallel zur Körperlängsachse ausgerichtetem Bein erfasst. Die gewonnenen Positionsdaten geben Hinweise auf eine etwaige Beinverlängerung bzw. Beinverkürzung sowie auch auf die sogenannte Lateralisierung bzw. Medialisierung, d.h. die "seitliche" Position des Femurs. Bei Hinweisen auf eine zu starke Medialisierung (Nach-Innen-Verschiebung) kann gegebenenfalls gegenüber dem Testschaft ein anderer Schaft in Verbindung mit einer anderen Kugel eingesetzt werden; in jedem Falle geben die Messwerte dem Arzt aber Hinweise, die bei der weiteren Versorgung des Patienten zu berücksichtigen sind.

Die nachfolgenden Anmerkungen beziehen sich auf eine Nutzung des beschriebenen Systems bei der Schaftpräparation und Implantation.

Für die Platzierung des Schaftes einer Hüftprothese ist die Herstellung eines geplanten Antetorsionswinkel des Femurhalses und die Winkelherstellung der ursprünglichen Beinlänge gefordert. Die Achsen-Ausrichtung des Schaftes richtet sich weitestgehend nach der Lage des Markkanals im Femur. Dies hat zur Folge, dass erst hieraus die eigentliche Schaftgröße bzw. dessen Offsets berechnet werden können.

Mit Hilfe einer kalibrierten Ahle wird der Markkanal des Femurs bestimmt. Eine weitere wichtige Information für die Platzierung des Schaftes ist die Bestimmung des Rotationszentrums; siehe dazu weiter oben.

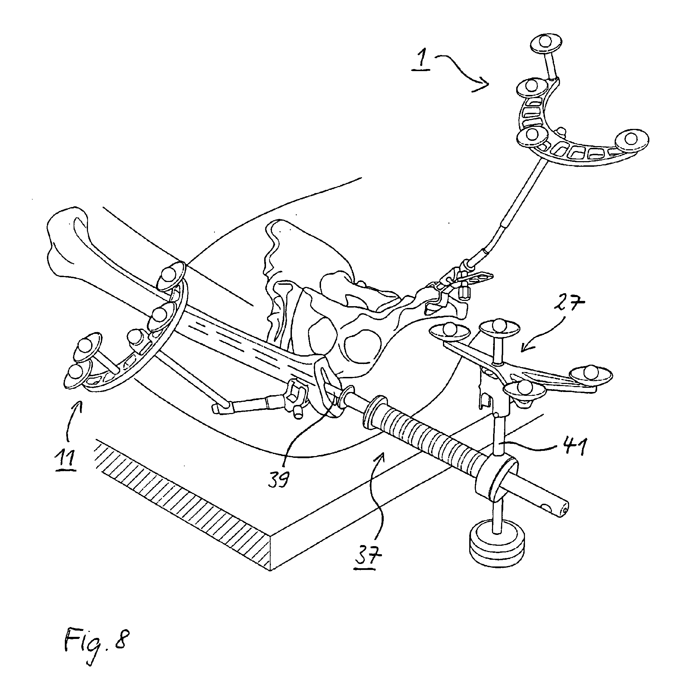

Fig. 8 zeigt eine hierzu einsetzbare weitere Komponente der vorgeschlagenen Anordnung, nämlich eine Markkanalahlen-Lokator-Kombination 37 mit einer Markkanalahle 39, einem Ahlenadapter 41 und (wiederum) einem Lokator 27, ähnlich der bereits in Fig. 3 gezeigten Lokatorausführung. Zum Einsetzen dieses Navigationsinstrumentes wird das proximale Femurende mit einem Kastenmeißel oder einer Stichsäge in der Nähe des Trochanter Major eröffnet und die Markkanalahle 39 dort von proximal eingeführt.

Präoperativ werden Inklinationswinkel und Antetorsionswinkel des Femurkopfes im Röntgenbild bestimmt und intraoperativ eingegeben. Zusätzlich ist die intraoperative Bestimmung des Antetorsionswinkel durch Messung von Landmarks am Kniegelenk und an dem oberen Sprunggelenk möglich, womit intraoperativ die Körperebenen bekannt sind. Die tatsächlichen Implantationswinkel und -positionen der Pfannennavigation können bei der Schaftimplantation mitberücksichtigt werden. Die letzte räumliche Position der Pfanne kann als relative Korrektur des Schaftes angebracht werden. Durch dieses Vorgehen ist eine optimale Implantation sichergestellt.

Die Präparation des Femurs zum Einsetzen des Schaftes erfolgt dann - analog zur Präparation des Pfannensitzes mit einem navigierten Fräser - mit einer navigierten Schaftraspel, d.h. einer Schaftraspel-Lokator-Kombination, welche der in Fig. 8 gezeigten Kombination sehr ähnlich ist und daher hier weder gezeigt noch genauer beschrieben wird. Nach der Präparation wird wiederum ein Testschaft eingesetzt und es werden die oben im Zusammenhang mit der pfannenseitigen Navigation beschriebenen Überprüfungen ausgeführt. Bei zufriedenstellenden Ergebnissen wird der endgültige Schaft dann eingesetzt, ohne dass dieser nochmals navigiert werden müsste.

Die Ausführung der Erfindung ist nicht auf die oben beschriebene Anordnung und das in Verbindung hiermit skizzierte Vorgehen beschränkt, sondern ebenso in Abwandlungen möglich, die im Rahmen fachgemäßen Handelns liegen.

### Bezugszeichenliste

- 1: Beckenkamm-Lokator
- 1.1: Grundkörper
- 1.2: Steckhülse
- 1.3: 4-Punkt-Lokatorfeld
- 3: Befestigungsklammer
- 3.1: Mediales Klammerteil
- 3.2: Laterales Klammerteil
- 5: Inbusschraube
- 7; 7': Handtaster
- 7.1: Taster-Grundkörper
- 7.2: 4-Punkt-Lokatorfeld
- 9: Tasterteil
- 9.1: Halter
- 11: Femur-Lokator
- 13: Femurklammer
- 13.1: erstes Grundteil
- 13.2: Zapfen
- 13.3: zweites Grundteil
- 15: Lokatorstab
- 15.1: Steckhülse
- 17: Halbkugel
- 19: Kugeladapter-Handtaster-Kombination
- 21: Fräser-Lokator-Kombination
- 23: Fräswelle
- 25: Fräswellenadapter
- 27: Lokator
- 29: Setzinstrument-Lokator-Kombination
- 31: Setzinstrumentenschaft
- 33: Schaftadapter
- 35: Hüftpfanne
- 37: Markkanalahlen-Lokator-Kombination
- 39: Markkanalahle
- 41: Ahlenadapter

## Patentansprüche

1. Anordnung zur Ermittlung funktionsbestimmender geometrischer Größen eines Gelenkes eines Wirbeltiers, insbesondere eines Hüft- oder Schultergelenkes eines Menschen, zur Vorbereitung des Einsetzens eines Gelenkersatzimplantats, insbesondere einer Hüft- oder Schultergelenkpfanne oder eines zugehörigen Schaftimplantats, mittels eines optischen Koordinatenmessverfahrens, mit
- einer Stereokamera oder -kameranordnung zur räumlichen Erfassung optischer Gebersignale,
- einem mobilen Mehrpunktgeber (7, 37), der als beweglicher Taster zum Antasten knöcherner Referenzen im Gelenkbereich zur Bestimmung von deren Koordinaten ausgebildet ist,
- einem skelettfesten Mehrpunktgeber (1, 3), der zur starren Befestigung, insbesondere zum Anschrauben oder Anklemmen, an einer vom dem Gelenk ausgehenden Extremität, insbesondere nahe dem maximalen Ende eines Femurs oder eines Humerus, ausgebildet ist,
- einer interaktiven Ablaufsteuerung zur Steuerung der sequentiellen Registrierung und Abspeicherung von einer durch den mobilen Mehrpunktgeber gelieferten Messpunkt koordinatenmengen und Vm in einer ersten Mehrzahl von Positionen des skelettfesten Mehrpunktgebers in einer Mehrzahl von Drehstellungen der Extremität erfassten Messpunktkoordinatenmengen und deren anschließenden Verarbeitung nach einem vorgespeicherten Verarbeitungsablauf,
- einer Auswertungseinheit zur Auswertung der durch die Mehrpunktgeber gelieferten und die Kameraanordnung erfassten Messpunktkoordinatenmengen zwecks Bestimmung der geometrischen Größen, die Mittel zur Bestimmung der transversalen, vertikalen und sagittalen Körperachsen sowie Mittel zur Ausführung eines iterativen Verfahrens, insbesondere einer Ausgleichungsrechnung nach der Methode der kleinsten Quadrate, zur Bestimmung der Koordinaten des Rotationszentrums des Gelenkes umfasst, und
- einer mit der Ablaufsteuerung sowie der Auswertungseinheit verbundenen Ausgabeeinheit (Fig. 6) zur Ausgabe von Handlungsaufforderungen an einen Operateur gemäß dem vorbestimmten Verfahrensablauf und in Abhängigkeit von den Ergebnissen der Bestimmung der geometrischen Größen sowie zur Anzeige der Auswertungsergebnisse.

2. Anordnung nach Anspruch 1,
**gekennzeichnet durch**
einen weiteren skelettfesten Mehrpunktgeber (11, 13), der zur starren Befestigung, insbesondere zum Anschrauben oder Anklemmen, an einem knöchernen pfannenseitigen Bereich des Gelenkes, insbesondere an einem Beckenkamm oder am Pfannendach, ausgebildet ist, wobei
die interaktive Ablaufsteuerung auch zur Steuerung der Registrierung und Abspeicherung einer **durch** den weiteren skelettfesten Mehrpunktgeber gelieferten Messpunktkoordinatenmenge und die Auswertungseinheit zur Auswertung derselben ausgebildet sind.

3. Anordnung nach Anspruch 1 oder 2,
**dadurch gekennzeichnet, dass**
die Ausgabeeinheit (Fig. 6) zur Anzeige der Auswertungsergebnisse in grafischer Darstellung, insbesondere in einer synoptischen bildlichen Darstellung mit einem durch ein bildgebendes Untersuchungsverfahren gewonnenen zwei- oder dreidimensionalen Abbild des Gelenkbereiches, ausgebildet ist.

4. Anordnung nach einem der vorangehenden Ansprüche,
**gekennzeichnet durch**
eine erste und zweite verstellbare Klemmvorrichtung (3, 13) als Adapter zur Fixierung des ersten und zweiten skelettfesten Mehrpunktgebers an dem Gelenk bzw. an der Extremität.

5. Anordnung nach einem der vorangehenden Ansprüche,
**dadurch gekennzeichnet, dass**
- ein dritter skelettfester Mehrpunktgeber zur im wesentlichen starren Befestigung, insbesondere mittels einer verstellbaren Manschette, an einer zweiten Extremität vorgesehen ist, die von einem nicht zu operierenden zweiten Hüft- oder Schultergelenk ausgeht, und
- die Auswertungseinheit zur Bestimmung von geometrischen Größen des zweiten Hüft- bzw. Schultergelenkes als Referenz für die geometrischen Größen des ersten Gelenkes ausgebildet ist.

6. Anordnung nach einem der vorangehenden Ansprüche,
**dadurch gekennzeichnet, dass**
- der mobile Mehrpunktgeber (1, 3) zum äußeren Antasten von knöchernen Referenzen an der von dem zu ersetzenden Gelenk und wahlweise dem zweiten Hüft- oder Schultergelenk ausgehenden zweiten Extremität ausgebildet oder hierzu ein weiterer, als beweglicher Taster ausgebildeter Mehrpunktgeber zum Antasten derartiger Referenzen vorgesehen und
- die Auswertungseinheit zur Auswertung der Messpunktkoordinaten dieser knöchernen Referenzen zur Bestimmung mindestens einer der geometrischen Größen, insbesondere der Länge der Extremität, ausgebildet ist.

7. Anordnung nach einem der vorangehenden Ansprüche,
**gekennzeichnet durch**
- ein Resektionsinstrument (23), insbesondere einen Fräser oder eine Raspel, das mit dem mobilen oder einem weiteren Mehrpunktgeber starr zu einer geometrisch kalibrierten, navigierbaren Werkzeug-Geber-Einheit (21) verbindbar ist, derart, dass aus den Gebersignalen dieser Einheit Positionskoordinaten eines Wirkabschnitts des Resektionsinstruments, insbesondere eines Fräserkopfes bzw. Raspelabschnittes, und hieraus wahlweise Positionskoordinaten eines mit dem Resektionsinstrument geschaffenen Resektionsabschnittes bestimmbar sind,
- eine Ausbildung einer Eingabeschnittstelle zur Eingabe von Instrumentparametern des Resektionsinstrumentes.

8. Anordnung nach einem der vorangehenden Ansprüche,
**gekennzeichnet durch**
- ein navigierbares Setzinstrument (31) das mit dem mobilen oder weiteren Mehrpunktgeber starr zu einer geometrisch kalibrierten Werkzeug-Geber-Einheit (29) verbindbar ist, derart, dass aus den Gebersignalen dieser Einheit Positionskoordinaten eines Wirkabschnitts des Setzinstrumentes bestimmbar sind,
- eine Ausbildung einer Eingabeschnittstelle zur Eingabe von Werkzeugparametern des Setzwerkzeuges.

9. Anordnung nach einem der vorangehenden Ansprüche,
**gekennzeichnet durch**
- eine Sonde, insbesondere Markkanalahle (39), zur Sondierung eines Markkanals der von dem Gelenk ausgehenden Extremität, die mit dem mobilen ten oder einem weiteren Mehrpunktgeber starr zu einer geometrisch kalibrierten, navigierbaren Sonden-Geber-Einheit (37) verbindbar ist, derart, dass aus den Geber-Signalen dieser Einheit die Lage der Markkanalachse bestimmbar ist, und
- eine Ausbildung einer Eingabeschnittstelle zur Eingabe von Sondenparametern.

10. Anordnung nach einem der Ansprüche 1 bis 9,
**dadurch gekennzeichnet, dass**
die Ablaüfsteuerung Mittel zur Menüführung unter Einschluss eines Eingabeschrittes zur Tastatur- oder Spracheingabe definierter realer oder virtueller Punkte des Gelenkbereiches und/oder von Lagebeziehungsvektoren zwischen solchen Punkten innerhalb des Gelenkbereiches oder von diesen zu gelenkfunktionsrelevanten Punkten an der Extremität außerhalb des Gelenkbereiches oder zur Datenübernahme entsprechender Daten eines dreidimensionalen Abbildes aus einem Auswertungsprogramm einer bildgebenden Untersuchung und eines Anzeigeschrittes eines Planungsergebnisses aufweist.

## Claims

1. Arrangement for the examination of function-determining geometric parameters of a joint in a vertebrate, in particular, a human hip or shoulder joint, in preparation for the insertion of a joint replacement implant, in particular, an acetabular or glenoid implant or an associated shaft implant, by means of an optical coordinate-measurement technique, comprising:
- a stereo camera or camera arrangement for the spatial registration of optical sensor signals,
- a mobile multi-point sensor (7; 37), which is designed as a mobile sensor for sensing bony references in the joint region in order to determine their coordinates;
- a skeletally-fixed multi-point sensor (1, 3), which is designed for rigid attachment, in particular, by screwing or clamping, to an extremity extending from the joint, in particular, close to the proximal end of a femur or a humerus;
- an interactive process-control unit for controlling the sequential registration and storage of a quantity of measuring-point coordinates delivered by the mobile multi-point sensor and of quantities of multi-point coordinates registered in a first plurality of positions of the skeletally-fixed multi-point sensor in a plurality of rotational positions of the extremity and their subsequent processing according to a previously-stored processing procedure;
- an evaluation unit for evaluating the quantities of multi-point coordinates delivered by the multi-point sensors and registered by the camera arrangement for the purpose of determining the geometrical parameters, which comprises means for determining the transversal, vertical and sagittal body axes and means for the execution of an iterative process, in particular, an equalisation calculation according to the method of least squares, in order to determine the coordinates of the rotational centre of the joint; and
- an output unit (Figure 6) connected to the process-control unit and the evaluation unit for the display of action requirements to an operator according to the predetermined processing procedure and dependent upon the results of the determination of the geometric parameters, and for the display of the evaluation results.

2. Arrangement according to claim 1,
**characterised by**
a further skeletally-fixed multi-point sensor (11, 13), which is designed for rigid attachment, in particular, by screwing or clamping, to a bony, cavitary region of the joint, in particular to an iliac crest or an acetabular roof, wherein the interactive process-control unit is also designed to control the registration and storage of a quantity of measuring-point coordinates delivered by the further skeletally-fixed multi-point sensor, and the evaluation unit is designed for the evaluation of the same.

3. Arrangement according to claim 1 or 2,
**characterised in that**
the output unit (Figure 6) is designed to display the evaluation results in a graphic display, in particular, in a synoptic-image display with a two-dimensional or three-dimensional image of the joint region obtained through an image-providing investigative technique.

4. Arrangement according to any one of the preceding claims,
**characterised by**
a first and second adjustable clamping device (3, 13) as an adapter for fixing the first and second skeletally-fixed multi-point sensor to the joint or respectively to the extremity.

5. Arrangement according to any one of the preceding claims,
**characterised in that**
- a third skeletally-fixed multi-point sensor is provided for substantially-rigid attachment, in particular, by means of an adjustable cuff, to a second extremity, which extends from a second hip or shoulder joint, which is not be operated; and
- the evaluation unit is designed for the determination of geometrical parameters of the second hip or respectively shoulder joint as a reference for the geometric parameters of the first joint.

6. Arrangement according to any one of the preceding claims,
**characterised in that**
- the mobile, multi-point sensor (1, 3) is designed for the external sensing of bony references on the second extremity extending from the joint to be replaced and optionally from the second hip or shoulder joint, or that a further multi-point sensor designed as a mobile sensor for sensing references of this kind is provided for this purpose; and
- the evaluation unit for evaluating the measuring-point coordinates of these bony references is designed for the determination of at least one of the geometric parameters, in particular, the length of the extremity.

7. Arrangement according to any one of the preceding claims,
**characterised by**
- a resection instrument (23), in particular, a milling or rasping device, which can be rigidly connected with the mobile multi-point sensor or with a further multi-point sensor to a geometrically-calibrated, navigable tool-sensor unit (21), in such a manner that position coordinates of an active portion of the resection instrument, in particular, of a milling head or a rasp portion can be determined from the sensor signals of this unit, and, optionally, position coordinates of a resection portion created with the resection instrument can be determined from the latter;
- a formation of an input interface for the input of instrument parameters of the resection instrument.

8. Arrangement according to any one of the preceding claims,
**characterised by**
- a navigable setting instrument (31), which can be rigidly connected with the mobile multi-point sensor or further multi-point sensor to a geometrically-calibrated tool-sensor unit (29) in such a manner that position coordinates of an active portion of the setting instrument can be determined from the sensor signals of this unit;
- a formation of an input interface for the input of tool parameters of the setting tool.

9. Arrangement according to any one of the preceding claims,
**characterised by**
- a probe, in particular, a medullary-canal awl (39) for probing a medullary canal of the extremity extending from the joint, which can be rigidly connected with the mobile multi-point sensor or a further multi-point sensor to a geometrically-calibrated, navigable probe-sensor unit (37) in such a manner that the position of the axis of the medullary canal can be determined from the sensor signals of this unit; and
- a formation of an input interface for the input of probe parameters.

10. Arrangement according to any one of claims 1 to 9,
**characterised in that**
the process-control unit provides means for menu control including an input stage for the input via keyboard or phonetic input of defined, real or virtual points of the joint region, and/or of relative-position vectors between such points within the joint region, or from the latter to points relevant to joint function on the extremity outside the joint region, or for the data transfer of corresponding data of a three-dimensional image from an evaluation program of an image-providing investigation, and including a display stage for a planning result.

## Revendications

1. Système permettant de déterminer des grandeurs géométriques définissant la fonction d'une articulation d'un vertébré, en particulier d'une articulation de hanche ou d'épaule d'un être humain, en préparation à la mise en place d'une prothèse d'articulation, en particulier d'une cavité acétabulaire d'articulation de la hanche ou d'une cavité glénoïdale d'articulation de l'épaule, ou d'un implant à tige correspondant, au moyen d'un procédé optique de mesure de coordonnées, comprenant
- une caméra stéréo ou un ensemble caméra stéréo servant à l'acquisition de signaux de transmetteurs optiques dans l'espace ;
- un transmetteur multipoints mobile (7, 37), réalisé sous la forme d'un palpeur déplaçable, servant à explorer des références osseuses dans la zone de l'articulation afin de déterminer leurs coordonnées ;
- un transmetteur multipoints solidaire du squelette (1, 3) conçu pour se fixer rigidement, en particulier par vissage ou bridage, à une extrémité partant de l'articulation, en particulier au voisinage de l'extrémité maximale d'un fémur ou d'un humérus ;
- une commande séquentielle interactive servant à commander la séquence d'enregistrement et de mémorisation de quantités de coordonnées de points de mesure fournies par le transmetteur multipoints mobile et de quantités de coordonnées de points de mesure relevées dans une première pluralité de positions du transmetteur multipoints solidaire du squelette dans une pluralité de positions en rotation de l'extrémité et ensuite à traiter ces données suivant un processus de traitement préalablement mémorisé ;
- un module d'exploitation de données, servant à analyser les quantités de coordonnées de points de mesure fournies par le transmetteur multipoints et acquises par l'ensemble caméra en vue de la détermination des grandeurs géométriques, qui comprend des moyens permettant de déterminer les axes transversal, vertical et sagittal du corps ainsi que des moyens pour exécuter un processus itératif, en particulier un calcul d'ajustement suivant la méthode des moindres carrés, permettant de déterminer les coordonnées du centre de rotation de l'articulation ; et
- un module de sortie (figure 6), relié à la commande séquentielle ainsi qu'au module d'exploitation de données, servant à délivrer à un opérateur des instructions d'actions suivant les séquences prédéterminées du procédé et en fonction des résultats de la détermination des grandeurs géométriques ainsi qu'à afficher les résultats de l'exploitation des données.

2. Système selon la revendication 1, **caractérisé par** un autre transmetteur multipoints solidaire du squelette (11, 13) conçu pour se fixer rigidement, en particulier par vissage ou bridage, sur une zone osseuse du côté de la cavité acétabulaire ou glénoïdale de l'articulation, en particulier à une crête iliaque ou au sourcil acétabulaire ou glénoïdal, la commande séquentielle interactive étant également conçue pour commander l'enregistrement et la mémorisation d'une quantité de coordonnées de points de mesure fournie par ledit autre transmetteur multipoints solidaire du squelette et le module d'exploitation de données étant conçu pour exploiter ces coordonnées.

3. Système selon la revendication 1 ou la revendication 2, **caractérisé en ce que** le module de sortie (figure 6) est conçu pour afficher les résultats de l'exploitation des données sous une forme graphique, en particulier sous la forme d'une représentation imagée synoptique, avec une reproduction en deux ou trois dimensions de la zone de l'articulation obtenue par un procédé d'analyse générant des images.

4. Système selon l'une des revendications précédentes, **caractérisé par** un premier et un deuxième dispositif de serrage réglable (3, 13) servant d'adaptateurs de fixation du premier et du deuxième transmetteur multipoints solidaire du squelette à l'articulation ou à l'extrémité.

5. Système selon l'une des revendications précédentes, **caractérisé en ce que**
- il est prévu un troisième transmetteur multipoints solidaire du squelette, conçu pour se fixer de manière sensiblement rigide, en particulier au moyen d'une manchette réglable, à une deuxième extrémité partant d'une deuxième articulation de hanche ou d'épaule non concernée par l'opération ; et
- le module d'exploitation de données est conçu pour déterminer des grandeurs géométriques de la deuxième articulation de hanche ou d'épaule en tant que référence pour les grandeurs géométriques de la première articulation.

6. Système selon l'une des revendications précédentes, **caractérisé en ce que**
- le transmetteur multipoints mobile (1, 3) est conçu pour explorer de l'extérieur des références osseuses sur l'extrémité partant de l'articulation à remplacer et au choix sur la deuxième extrémité partant de la deuxième articulation de hanche ou d'épaule ou bien il est prévu pour cela un autre transmetteur multipoints réalisé sous la forme d'un palpeur déplaçable afin d'explorer des références de ce genre ; et
- le module d'exploitation de données est conçu pour exploiter les coordonnées de points de mesure de ces références osseuses afin de déterminer l'une au moins des grandeurs géométriques, en particulier la longueur de l'extrémité.

7. Système selon l'une des revendications précédentes, **caractérisé par**
- un instrument de résection (23), en particulier une fraise ou une rugine, qui peut être relié rigidement au transmetteur multipoints mobile ou à un autre transmetteur multipoints pour former un ensemble outil/transmetteur orientable géométriquement calibré (21) de telle façon que les signaux de transmetteur provenant de cet ensemble permettent de déterminer des coordonnées de position d'un segment actif de l'instrument de résection, en particulier d'une tête de fraise ou d'un segment de rugine, et le cas échéant, à partir de celles-ci, des coordonnées de position d'un segment de résection créé au moyen de l'instrument de résection ;
- une représentation d'une interface de saisie permettant de saisir des paramètres de l'instrument de résection.

8. Système selon l'une des revendications précédentes, **caractérisé par**
- un instrument de placement orientable (31) qui peut être relié rigidement au transmetteur multipoints mobile ou à un autre transmetteur multipoints pour former un ensemble outil/transmetteur (29) de telle façon que les signaux de transmetteur provenant de cet ensemble permettent de déterminer des coordonnées de position d'un segment actif de l'instrument de placement ;
- une représentation d'une interface de saisie permettant de saisir des paramètres de l'outil de placement.

9. Système selon l'une des revendications précédentes, **caractérisé par**
- une sonde, en particulier un alésoir de canal médullaire (39), servant à sonder un canal médullaire de l'extrémité partant de l'articulation, qui peut être reliée rigidement au transmetteur multipoints mobile ou à un autre transmetteur multipoints pour former un ensemble sonde/transmetteur orientable géométriquement calibré (37) de telle façon que les signaux de transmetteur provenant de cet ensemble permettent de déterminer la position de l'axe du canal médullaire ; et
- une représentation d'une interface de saisie permettant de saisir des paramètres de la sonde.

10. Système selon l'une des revendications 1 à 9, **caractérisé en ce que** la commande séquentielle présente des moyens de pilotage par menus incluant une étape de saisie au clavier ou de saisie vocale de points réels ou virtuels de la zone de l'articulation et/ou de vecteurs de relation positionnelle entre ces points à l'intérieur de la zone de l'articulation ou entre ces points et des points concernant la fonction articulaire sur l'extrémité à l'extérieur de la zone de l'articulation, ou une étape de chargement de données correspondantes d'une représentation en trois dimensions provenant d'un programme d'exploitation de données d'une analyse générant des images, et incluant une étape de visualisation d'un résultat de planification.
